(19) Europäisches Patentamt
European Patent Office
Office européen des brevets

(11) Publication number: **0 033 233 A1**

(12) **EUROPEAN PATENT APPLICATION**

(21) Application number: 81300303.5

(22) Date of filing: 23.01.81

(51) Int. Cl.³: C 07 F 1/02
C 07 C 51/353

(30) Priority: 25.01.80 GB 8002609

(43) Date of publication of application: 05.08.81 Bulletin 81/31

(84) Designated Contracting States: AT BE CH DE FR GB IT LI LU NL SE

(71) Applicant: STERWIN AG.
Zeughausgasse 9
CH-6300 Zug(CH)

(72) Inventor: Peel, Richard
The Old School House Seaton Delaval
Northumberland NE25 OP5(GB)

(74) Representative: Green, Alan James et al,
Sanderson & Co. 97 High Street
Colchester Essex(GB)

(54) **A process for preparing alpha-lithio derivatives, derivatives whenever prepared thereby, a process for preparing carboxylic acids using them, and carboxylic acids whenever prepared thereby.**

(57) The present invention relates to a process for preparing α-lithio derivatives of the general formula:

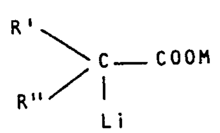

(1)

wherein R′ and R″, which may be the same or different, each represent a hydrogen atom, an alkyl, alkoxyalkyl, dialkylaminoalkyl, aralkyl, alkoxy or dialkylamino group, or a monocyclic aryl ring optionally substituted by one or more alkyl, alkoxy, dialkylamino or monocyclic aryl groups and M represents an alkali metal atom, and to their use. In the process of the invention an acid of the general formula:

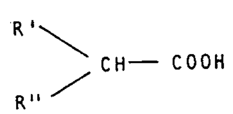

(11)

wherein R′ and R″ are as defined above or a salt thereof is reacted with lithium and a dialkylamine in the presence of an aromatic polynuclear hydrocarbon or an unsaturated hydrocarbon comprising an aliphatic or alicyclic chain containing a double bond conjugated either with a second double bond in the chain or with an aromatic moiety. The reactants are brought together so that there is substantially no lithium dialkylamide formed before the acid is introduced.

The thus formed derivatives can be reacted with a compound A-X where A is an alkyl or alkenyl group and X is halogen or $-O-SO_2-R^3$ where $R^3$ is alkyl or aryl to form an acid of the general formula:

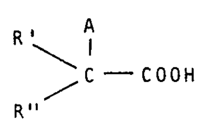

(111)

where R′ and R″ are as defined above.

EP 0 033 233 A1

"A Process for Preparing α-Lithio Derivatives, Derivatives Whenever Prepared Thereby, a Process for Preparing Carboxylic Acids Using Them, and Carboxylic Acids Whenever Prepared Thereby."

This invention relates to a process for preparing α-substituted organic carboxylic acids. In particular it concerns a new process for preparing α-lithio derivatives of organic carboxylic acids, which may thereafter be reacted to replace the lithium atom by an organic group such as an allyl or alkyl group.

In the past α-substitution of carboxylic acids has been carried out by a multi-step process involving formation of a lithium di-alkylamide, reaction of the lithium dialkylamide with the carboxylic acid to form an α-lithio derivative, and finally formation of the desired α-substituted product by reaction with an organic halide. The lithium dialkylamide used in such processes is typically prepared by metallation of the appropriate amine using an organic lithium compound such as phenyllithium or butyllithium, or directly from lithium and the appropriate amine in the presence of hexamethylphosphoric triamide. Neither of these techniques is entirely satisfactory. The organic lithium compounds are extremely difficult and dangerous to handle as they are spontaneously inflammable, while hexamethylphosphoric triamide is both expensive and carcinogenic. We have also found that the traditional Ziegler process for preparing lithium alkylamides, by reacting lithium and an amine in the presence of a conjugated unsaturated hydrocarbon, gives poor and highly impure yields of dialkylamides.

The prior art process may be used to form an α-lithio

derivative of an organic carboxylic acid only slowly and at the expense of considerable quantities of the lithium and amine starting materials. Moreover, the yields of that prior art process are poor.

We have now established that α-lithio carboxylic acid salts may be prepared from lithium and acid in a single step, provided that the reactants are brought together in a particular manner. In this single step reaction a more rapid formation of the α-lithio compound has been noted, and the reaction appears to be more economic in that it has given good yields from reduced amounts of the starting materials. The use of dangerous starting materials is also avoided.

Thus, in one aspect this invention provides a process for preparing α-lithio derivatives of the general formula:

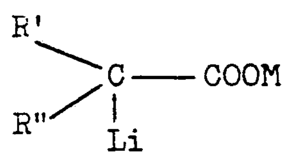

(I)

(wherein R' and R", which may be the same or different, each represent a hydrogen atom, an alkyl, alkoxyalkyl, dialkylaminoalkyl, aralkyl, alkoxy or dialkylamino group, or a monocyclic aryl ring optionally substituted by one or more alkyl, alkoxy, dialkylamino or monocyclic aryl groups and M represents an alkali metal atom), in which process an acid of the general formula:

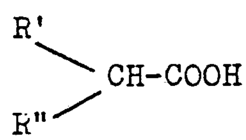

(II)

(wherein R' and R" are as defined herein) or a salt thereof is reacted with lithium and a dialkylamine in the presence of a proton acceptor which is an aromatic polynuclear hydrocarbon or an unsaturated hydrocarbon comprising an

aliphatic or alicyclic chain containing a double bond conjugated either with a second double bond in the chain or with an aromatic moiety, the reactants being brought together so that there is substantially no lithium dialkylamide formed before the acid is introduced.

When R' and/or R" represents an alkyl group, an alkoxy group, a dialkylamino group or an aryl ring substituted by an alkyl group, the alkyl moiety preferably contains from 1 to 10 carbon atoms.

M is preferably a lithium atom.

A particularly preferred starting material of general formula II is 2-ethyl butyric acid.

When the starting material of general formula II is in the form of a salt this is preferably a salt formed with lithium or a metal cation more electropositive than lithium, such as a sodium cation. When the compound II is in the form of an alkali metal salt other than the lithium salt the product is a compound I wherein M is other than lithium. It is also possible, however, to employ the compound of general formula II in the form of a secondary, tertiary or quaternary amine salt. The acid of general formula II could therefore be introduced in the form of the secondary amine salt formed with the dialkylamine participating in the reaction.

The dialkylamine used in the process of the invention is preferably an amine in which the alkyl groups contain from 1 to 10 carbon atoms. Particularly excellent results have been obtained with di*iso*propylamine, and accordingly the use of this amine is highly preferred. It has surprisingly been found that the dialkylamine acts catalytically in the process of the invention.

It is believed, although the invention is not limited by such theoretical considerations, that the reaction proceeds according to the following stoichiometric reactions

(wherein R represents an alkyl group):

$$R'R''CHCOOH + Li \longrightarrow R'R''CHCOOLi$$

$$R'R''CHCOOLi + R_2NH + Li \longrightarrow R'R''CLiCOOLi + R_2NH$$

Thus, it is possible to use relatively small amounts of the dialkylamine, and while from 0.1 to 20 moles (based on the acid II or its salt) could be used, the preferred range is from 0.5 to 1.0 moles. Excellent results have been obtained with 0.5 equivalents of the dialkylamine.

As in the Ziegler reaction, the process of the invention employs an aromatic polynuclear hydrocarbon or unsaturated hydrocarbon containing a conjugated double bond system which acts as a proton acceptor or hydrogen sink and together with the dialkylamine operates to introduce the α-lithium atom in the organic carboxylic acid. For convenience these "unsaturated conjugated hydrocarbons" are referred to herein as "proton acceptors", which term includes the aromatic, aliphatic and alicyclic hydrocarbons defined hereinbefore. Examples of suitable proton acceptors are naphthalene, styrene, cyclohexa-1,3--diene and butadiene. Naphthalene is especially preferred.

The proton acceptor is preferably present in an amount of from 0.5 to 1.5 moles per mole or organic carboxylic acid II or its salt, although greater amounts would cause no adverse effects and merely be wasteful and uneconomic. Very preferably from 0.9 to 1.3 moles, and most advantageously about 1.1 moles, of the proton acceptor hydrocarbon are used per mole of the acid II or its salt.

Lithium is preferably employed as the pure metal but may alternatively be used in the form of an alloy, particularly with another alkali metal such as sodium or potassium. Stoichiometrically 2 moles of lithium are required per mole of acid II, but it is generally advantageous to employ a small excess. Preferably from 1.9 to 2.5 moles of lithium are used per mole of the

acid II. When an alkali metal salt of the acid II is employed, the amount of lithium may be reduced as only one mole is stoichiometrically required.

The activity of the lithium may be increased by treating it with an agent to clean the metal surface. Such agents are well-known and include acids, alkyl halides and iodine. While a wide variety of acids may be employed, both organic and inorganic, it is obviously most convenient to use the acid of general formula II. To do this, a minor portion of the acid may be mixed with the lithium before this is added to the remainder of the reaction mixture.

It is highly advantageous to carry out the process of the invention in a solvent, and if a separate solvent is employed it is desirably polar and aprotic in the reaction mixture of the invention. Ethers are particularly suitable for use as solvents, and most preferably tetrahydrofuran (THF) is employed. In some systems it may not be necessary to employ a separate solvent, but instead to use the dialkylamine as a solvent for the other reactants.

The process of the invention effectively prepares the α-lithio derivative of an organic carboxylic acid in a single step, and this is achieved by bringing the reactants together so that the formation of lithium alkylamide is avoided before the acid is introduced. This means that the lithium, amine and proton acceptor must not be brought together before the acid is introduced into the reaction mixture.

The formation of lithium dialkylamides must be avoided, and to that end the acid of general formula II or its salt is added to the remainder of the reaction mixture either before or with the proton acceptor, but not after that proton acceptor has been incorporated in the reaction mixture.

The reaction is highly exothermic so that care is needed to keep the reaction under control. Very preferably the acid of general formula II (or its salt) and the proton acceptor are first mixed together, optionally with a solvent, and then the formed acid/proton acceptor mixture is mixed with the remaining reactants - that is, the lithium and amine, again optionally with a solvent. A modification of this mixing procedure occurs when, as described above, a minor portion of the acid is used to activate the lithium. In that case the mixing sequence is as follows:-

Li(+ solvent) + acid + dialkylamine → }
proton acceptor (+ solvent) + acid → } Reaction mixture

In some cases a lithium/dialkylamine/acid mixture results in a complex salt which is highly insoluble and hinders the reaction. In these cases it is desirable to mix substantially all the acid with the proton acceptor in the first instance. However, in the cases where the complex is not formed or is not insoluble, a different sequence of mixing may be used in which: firstly, the lithium, amine and acid are admixed optionally with a solvent; and secondly, the proton acceptor is added, optionally in a solvent. This sequence is therefore:-

Li + amine + acid (+ solvent) → }
proton acceptor (+ solvent) → } Reaction mixture

If the acid of general formula II is used in the form of a metal salt (other than a lithium salt) this alternative sequence may be modified by adding the lithium with the proton acceptor. Thus, the following sequence may be adopted:-

salt (+ solvent) + dialkylamine → }
proton acceptor (+ solvent) + lithium → } Reaction mixture

If a salt is used rather than the free acid this may be formed in an initial step from the free acid by

well-known salt-forming techniques. For example, the free acid may be reacted with an alkali metal hydride to form the corresponding alkali metal salt. Since salts other than alkali metal salts will immediately be converted to lithium salts in the reaction mixture of the process of the invention, there is little or no advantage to be gained from employing salts other than those of alkali metals.

The reaction mixture having been formed, best results are obtained when the mixture is agitated, e.g. stirred, or otherwise mixed, so that the lithium is in intimate contact with the other reactants, i.e. does not merely bob on the surface of the liquid remainder of the reaction mixture.

The reaction temperature preferably is just below the boiling point of the reaction mixture, but may be as low as ambient temperature. The preferred upper limit should ensure that the reactive lithium, which creates a hot spot, remains in contact with liquid, rather than surrounding itself with e.g. solvent, vapour.

The reaction temperature can be controlled by cooling or by varying the rate at which various reactants are added, for example, by varying the rate of addition of proton acceptor (+ solvent) and acid to Li (+ solvent) + acid + dialkylamine.

Having formed the lithio derivative of general formula I, this may thereafter be reacted to produce α-substituted carboxylic acids.

Thus, this invention also provides a process for preparing carboxylic acids of the general formula:

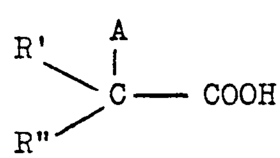

(III)

(wherein R' and R" are as defined hereinbefore and A represents an alkyl or alkenyl group), in which process a compound of general formula I is reacted with a compound of the general formula:

$$A —— X \qquad (IV)$$

(wherein A is as defined herein and X represents a halogen atom or a sulphonate group $-O-SO_2-R^3$ in which $R^3$ represents an alkyl or aryl group.

The group A introduced into the α-position of the carboxylic acid is of course chosen having regard to the final product sought, but A is preferably an alkyl or allyl group and the allyl group is especially preferred.

The compound of general formula IV is preferably a chloride, and thus allyl chloride is a particularly preferred organic halide IV. When X represents a sulphonate group, $R^3$ is preferably a methyl, phenyl or p-toluyl group.

The compound IV is stoichiometrically required in an amount equivalent to the α-lithio derivative of general formula I. On the grounds of economy a substantilly equivalent amount is preferably employed, although a small excess may improve the quality of the product.

The formation of the α-substituted product of general formula III is preferably carried out in a solvent, and those described hereinbefore in relation to the formation of the α-lithio derivative I are preferred. It is most convenient to carry out the formation of the product III without first isolating the α-lithio derivative I, and thus by simply adding the appropriate compound IV to the reaction mixture containing the formed α-lithio derivative I.

Work-up of the formed product III may be carried out by standard techniques within the competence of one skilled in the art. For example, any solvent and

dialkylamine may be removed by distillation, and the residue purified by separation between immiscible organic and aqueous solvents, such as isopropyl acetate and water. The volatile organic matter may be driven off from the aqueous component by steam distillation, and the purified product isolated by acidification of the aqueous layer.

The process of the invention may be extended to the formation of di-(α-substituted)-carboxylic acids. Following formation of an α-substituted compound of general formula III which also falls within general formula II - i.e. possesses a replaceable α-hydrogen atom - the procedure may be repeated to form a α-lithio derivative, which is reacted with a compound IV to form a product further substituted at the α-position.

The processes of the invention may be used to prepare a wide variety of organic compounds having utility in many different fields. By way of example only, it is pointed out that ethylbutyric acid may be converted to its corresponding α-lithio derivative and reacted with allyl chloride to form diethylpentenoic acid which is useful as an intermediate.

The following Examples are now given. though only by way of illustration, to show in detail certain aspects of the invention.

Example 1: Preparation of 2,2-diethyl-pent-4-enoic acid.

36 g of lithium and 700 ml of tetrahydrofuran were charged into a flask and the apparatus flushed with nitrogen. To this flask was charged, by a dropping funnel, 15 g of 2-ethylbutyric acid. The mixture was stirred for 15 minutes. 240 g of di*iso*propylamine were then charged by the dropping funnel.

300 g of naphthalene, 276 g of 2-ethylbutyric acid and 700 ml of tetrahydrofuran were made into a solution. 20% of this solution was charged to the flask by means of the dropping funnel. An exothermic reaction started and the temperature of the contents of the flask rose to 60°C, going orange as they reached this temperature, the period of the reaction being 40 minutes.

The remaining naphthalene solution was added over $1^1/2$ hours, holding the temperature at from 50 to 55°C. The mixture was stirred at 50°C for 3 hours thereafter, by which time the lithium had dissolved.

The mixture was then cooled to 20°C and 208 g of allyl chloride were run in over 1 hour, holding the temperature below 40°C.

1400 ml of solvents were distilled off. Then *iso*propyl acetate and water were added, the layers allowed to separate, and the organic layer washed with more water. From the combined aqueous layers 2,2-diethyl-pent-4-enoic acid separated on acidification. Distillation of this crude acid gave a pure product in a 90% overall yield.

To provide a comparison the above procedure was also followed using preformed lithium di*iso*propylamide (after Ziegler, and thus not a process of the invention).

This gave no more than 70% yields of the desired product, and dissolution of the metal took twice as long even though THF was the solvent.

Example 2: Preparation of 2,2-diethyl-pent-4-enoic acid - pilot plant scale.

To a 454 l vessel was charged 80 l of THF and 4.8 kg of lithium metal as truncated cones. 2.2 kg of 2-ethylbutyric acid were added and, after stirring for 30 minutes, 25 l (17.9 kg) of diisopropylamine.

Then there were added 15 l of a mixture prepared from 60 l of THF, 34.5 kg of 2-ethylbutyric acid and 40.0 kg of naphthalene. The vessel was heated to 50°C and stirred until the mixture became orange after a 6 to 7 hour induction period which may be shortened by more efficient agitation. The remainder of the above mixture was charged over 3 hours, the temperature being held at from 50 to 60°C, then the whole was stirred for 10 hours at 50 to 60°C. The resultant mixture was then cooled to below 20°C and 26.0 kg of allyl chloride were charged over 2 hours, holding the temperature below 40°C.

The mixture was worked up by removal of most of the THF and diisopropylamine by distillation, separation of the residue between isopropyl acetate and water, steam distillation of volatile organics from the aqueous layer, followed by acidification of the aqueous layer to liberate the desired acid. A 95% crude yield of 2,2-diethyl-pent-4-enoic acid at 88% purity was obtained (4% starting material, other impurities were solvents).

Example 3: Preparation of 2,2-diethyl-pent-4-enoic acid - Alternative mixing procedure.

7.5 g of lithium, 55g of diisopropylamine, 58 g of 2-ethylbutyric acid and 200 ml of THF were placed in a flask. To this was added slowly 64 g of naphthalene in 200 ml of THF, holding the temperature at 40°C by means of an ice bath. The mixture was stirred at 40°C until the lithium had reacted, then reacted with allyl chloride and worked up as in Examples 1 and 2. A 90% yield of 2,2-diethyl-pent-4-enoic acid was obtained.

Example 4: Preparation of 2,2-diethyl-pent-4-enoic acid - using diethylamine.

The procedure of Example 3 was repeated, but using 40 g of diethylamine in place of the diisopropylamine. A 51% yield 2,2-diethyl-pent-4-enoic acid was obtained.

Example 5: Preparation of 2,2-diethyl-pent-4-enoic acid from sodium ethylbutyrate.

A slurry of sodium 2-ethylbutyrate was prepared from 58 g of 2-ethylbutyric acid, 18.5 g of sodium hydride, 200 ml of tetrahydrofuran and 64 g of diisopropylamine.

To this was charged 32 g of naphthalene, 100 ml of THF and 3.8 g of lithium shot. The whole was stirred for 17 hours at room temperature, then reacted with 42 g of allyl chloride and worked up similarly as in Example 1. GLC indicated a 78% yield of the desired product.

Example 6: α-Propylation of Propanoic Acid.

7.5 g of lithium was placed in a flask with 128 ml of tetrahydrofuran and 50 g of diisopropylamine. Approximately 20% of a solution of 64 g of naphthalene in 96 ml of THF and 30 g of propanoic acid were then added.

The mixture was stirred at reflux, under nitrogen, until the colour changed to yellow brown (after about 3 hours) when the remaining naphthalene solution was added over 2 hours, at the end of which most of the lithium had dissolved.

The solution was stirred at room temperature overnight.

63 g of 2-bromopropane was added and the whole heated to reflux for 4 hours.

Then 250 ml of water was charged, after which the aqueous and organic layers were separated. The organic layers were washed with 50 ml of water, the combined aqueous layers heated to reflux, and the distillate

removed until the boiling point reached 100°C, and the distillate ceased to be cloudy.

The residue was acidified to a pH of less than 2. 12 g of a brown oil separated. A further 4 g of product was extracted from the aqueous layer with isopropyl acetate. Both portions assayed by glc as about 15% propanoic acid and the remainder another component.

The component was identified, by conversion, through the acid chloride and treatment with aqueous ammonia, to an amide, m.p. 129°C. This melting point is identical to that of pure 2,3-dimethylbutanamide and thus the major product of the alkylation was 2,3-dimethylbutanoic acid.

CLAIMS

1. A process for preparing α-lithio derivatives of the general formula:

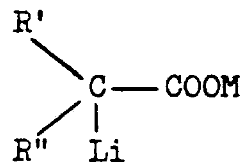

(I)

wherein R' and R", which may be the same or different, each represent a hydrogen atom, an alkyl, alkoxyalkyl, dialkylaminoalkyl, aralkyl, alkoxy or dialkylamino group, or a monocyclic aryl ring optionally substituted by one or more alkyl, alkoxy, dialkylamino or monocyclic aryl groups and M represents an alkali metal atom, in which process an acid of the general formula:

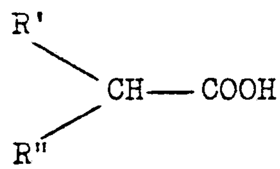

(II)

wherein R' and R" are as defined above or a salt thereof is reacted with lithium and a dialkylamine in the presence of a proton acceptor which is an aromatic polynuclear hydrocarbon or an unsaturated hydrocarbon comprising an aliphatic or alicyclic chain containing a double bond conjugated either with a second double bond in the chain or with an aromatic moiety, the reactants being brought together so that there is substantially no lithium dialkylamide formed before the acid is introduced.

2. A process as claimed in Claim 1 in which R' and/or R" represents an alkyl group, an alkoxy group, a dialkylamino group or an aryl ring substituted by an alkyl group and the alkyl moiety contains from 1 to 10 carbon atoms.

3. A process as claimed in Claim 1, in which the starting material of general formula II is 2-ethylbutyric acid.

4. A process as claimed in any of the preceding claims in which the alkyl groups in the dialkylamine contain from 1 to 10 carbon atoms.

5. A process as claimed in Claim 4, in which the dialkylamine is di*iso*propylamine.

6. A process as claimed in any of the preceding claims, in which from 0.5 to 1.0 moles of dialkylamine are employed per mole of the acid of general formula II or its salt.

7. A process as claimed in any of the preceding claims, in which the proton acceptor is naphthalene, styrene, cyclohexa-1,3-diene or butadiene.

8. A process as claimed in any of the preceding claims, in which the proton acceptor is present in an amount of from 0.5 to 1.5 moles per mole of the acid of general formula II or its salt.

9. A process as claimed in any of the preceding claims, in which from 1.9 to 2.5 moles of lithium are used per mole of the acid II.

10. A process as claimed in any of the preceding claims, which is carried out in a solvent which is polar and aprotic in the reaction mixture.

11. An α-lithio derivative of general formula I whenever prepared by a process as claimed in any of the preceding claims.

12. A process for preparing carboxylic acids of the general formula:

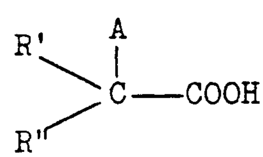

(III)

wherein R' and R" are as defined in Claim 1 and A represents an alkyl or alkenyl group, in which process a compound of general formula I as claimed in Claim 11

is reacted with a compound of the general formula:

$$A\text{-}X \qquad (IV)$$

wherein A is as defined above and X represents a halogen atom or a sulphonate group $-O-SO_2-R^3$ in which $R^3$ represents an alkyl or an aryl group.

13. A process as claimed in Claim 12, in which A represents an allyl group.

14. A process as claimed in Claim 12 or Claim 13 which is carried out in a solvent as claimed in Claim 10.

15. A carboxylic acid of general formula III whenever prepared by a process as claimed in any of Claims 12 to 14.

0033233

European Patent Office

# EUROPEAN SEARCH REPORT

Application number
EP 81 30 0303

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.3) |
|---|---|---|---|
| | JOURNAL OF THE AMERICAN CHEMICAL SOCIETY, vol. 89, no. 10, 10th May 1967, pages 2500-2501<br>P.L. CREGER: "Metalated carboxylic acids. I. Alkylation"<br>* Page 2500, left-hand column, last 2 lines - page 2501, left-hand column, second paragraph, line 8 *<br>-- | 1-15 | C 07 F 1/02<br>C 07 C 51/353 |
| | JOURNAL OF ORGANIC CHEMISTRY, vol. 35, no. 1, January 1970, pages 262-264<br>P.E. PFEFFER et al.: "α Anions of carboxylic acids. I. Effect of hexamethylphosphoramide on metalation and aklylation"<br>* Page 263, last paragraph, lines 1-5; page 264, paragraphs 1,2 *<br>-- | 1-15 | TECHNICAL FIELDS SEARCHED (Int. Cl.3)<br>C 07 F 1/00<br>1/02 |
| | US - A - 2 909 565 (DAVID O. DE PREE)<br>* Column 8, lines 47-62; column 6, lines 39-42; column 3, line 43 - column 4, line 51; column 7, line 53 - column 8, line 35 *<br>---- | 1-15 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 15-04-1981 | SUTER |

EPO Form 1503.1 06.78